(19) Europäisches Patentamt

European Patent Office

Office européen des brevets

(11) Veröffentlichungsnummer: **0 308 741 B1**

(12) **EUROPÄISCHE PATENTSCHRIFT**

(45) Veröffentlichungstag der Patentschrift: **26.05.93**

(21) Anmeldenummer: **88114731.8**

(22) Anmeldetag: **09.09.88**

(51) Int. Cl.5: **G01N 15/02**, G01N 15/06, G01N 21/85

(54) Kontrollverfahren bezüglich des Dispersionsgrades magnetischer Pigmente in einer Dispersion.

(30) Priorität: **22.09.87 DE 3731804**

(43) Veröffentlichungstag der Anmeldung:
**29.03.89 Patentblatt 89/13**

(45) Bekanntmachung des Hinweises auf die
Patenterteilung:
**26.05.93 Patentblatt 93/21**

(84) Benannte Vertragsstaaten:
**DE FR GB IT NL**

(56) Entgegenhaltungen:
**US–A– 3 690 771**
**US–A– 3 874 799**
**US–A– 4 449 821**

**FARBE + LACK, Band 88, Nr. 4, 1982, Seiten 251–253; G. GEISMAR: "Cr2O3–Pulver mit unterschiedlicher Teilchengrössenverteilung"**

**PATENT ABSTRACTS OF JAPAN, Band 7, Nr. 210 (P–223)[1355], 16. September 1983 & JP–A–58 103 071 (MATSUSHITA DENKO K.K.) 18–06–1983**

**DIN 53238 Teil 22 und DIN 53236 aus DIN**

Taschenbuch 49, Farbmittel 1, Berlin, 1985

(73) Patentinhaber: **BASF Magnetics GmbH**
**Gottlieb–Daimler–Strasse 10**
**W–6800 Mannheim(DE)**

(72) Erfinder: **Schulz, Horst, Dr.**
**Thalhamerstrasse 23**
**W–8160 Miesbach(DE)**

(74) Vertreter: **Münch, Volker et al**
**BASF Aktiengesellschaft Patentabteilung–C6 ZSP/A**
**W–6700 Ludwigshafen (DE)**

# Beschreibung

Die Erfindung betrifft ein Verfahren zur Kontrolle des Dispersionsgrads ferromagnetischer Pigmente beim Dispergieren.

Dispersionen, welche magnetische Pigmente enthalten, werden zur Herstellung von magnetischen Aufzeichnungsträgern beispielsweise Magnetplatten und Magnetbändern eingesetzt. Diese Dispersionen bestehen aus einem pulverförmigen magnetischen Pigment, einem Lösungsmittel oder Lösungsmittelgemisch, mindestens einem in diesem Lösungsmittel (Gemisch) löslichen oder dispergierbaren organischen Polymeren oder Präpolymeren, Dispergierhilfsmittel und weiteren organischen und/oder anorganischen Zusatzstoffen, wie Gleitmittel, Viskositätsregulatoren, Stabilisatoren, anorganische Substanzen zur Regulierung der Leitfähigkeit, des Abriebs, der Rauhigkeit und so weiter. Bei der Herstellung der Dispersionen werden im allgemeinen Verfahren der Lackherstellung angewandt. Dabei ist neben der präzisen Einhaltung der chemischen Zusammensetzung ein komplizierter mechanischer Bearbeitungsprozeß in Dispergiereinrichtungen, die weiter unten geschildert werden, von großer Wichtigkeit. Dieser und die physikalisch-chemischen Eigenschaften der Bestandteile der Dispersion sowie ihre gegenseitige Wirkung bestimmen die innere Struktur der Dispersion, wobei der Dispersionsgrad und der Grad der Agglomeration der einzelnen Teilchen, insbesondere der magnetischen Pigmente, eine entscheidende Rolle spielen. Elektrostatische und magnetostatische Kräfte zwischen den Pigmenten können zu Agglomerationserscheinungen führen, die erst später im fertigen magnetischen Aufzeichnungsträger sichtbar werden.

Wesentliche Eigenschaften für die Güte der Dispersion sind also der Dispersionsgrad beziehungsweise der Agglomerationsgrad, die Teilchendichte und die Viskosität. Darüber hinaus spielt die Stabilität der Dispersion eine Rolle, die stark von der Zeit abhängig ist, sich also in der Zeit von der Lackherstellung bis zum Beguß der Dispersion ändern kann. Üblicherweise geschieht die Herstellung der Dispersion in drei Schritten.

1. Das magnetische Pigment wird mit oder ohne Lösungsmittel in Gegenwart eines Netzmittels und gegebenenfalls eines gelösten polymeren Bindemittels vorgemahlen.

2. In der Vordispergierstufe wird der unter 1. beschriebene Pigmentniederschlag in eine konzentrierte Bindemittellösung hineindispergiert, worauf gegebenenfalls weitere der oben genannten Zusätze zugefügt werden.

3. In einem länger andauernden Feindispergierverfahren wird der erforderliche Dispersionsgrad der magnetischen Dispersion hergestellt.

Die Qualität der Dispersion wird zur Zeit in der Produktion weitgehend dadurch überprüft, daß ein Probebeguß hergestellt wird, der im wesentlichen einem fertigen magnetischen Aufzeichnungsträger entspricht und daß dann dessen Qualität überprüft wird. Von dieser Überprüfung wird die weitere Behandlung der Dispersion abhängig gemacht. Es ist einleuchtend, daß eine derartige Überprüfung zeitaufwendig, kostspielig und ungenau ist, da unmittelbar vor und während des Auftragens der Dispersion auf einem Träger keine Aussage über den Zustand der Dispersion möglich ist.

In der EP 0 146 015 ist eine schnelle Methode der Dispersionsgradbestimmung in strömenden Zweiphasensystemen beschrieben. Dabei wird der Druckabfall gemessen, den die Dispersion in einer definierten Laufstrecke erfährt. Diese Methode dürfte für den vorstehend genannten Zweck zu grob sein.

Aus der DE-PS 23 37 165 ist ein Verfahren zur Messung des elektrokinetischen Zeta-Potentials einer Dispersion bekannt. Aus der zu untersuchenden Dispersion wird kontinuierlich ein Probenstrom entnommen und in eine Trennzelle geschickt, in der der Probenstrom einem Magnetfeld ausgesetzt und in mehrere Teilströme zerlegt wird. Diese Teilströme werden dann durch einzelne separate Meßzellen geleitet, in denen der Feststoffgehalt der einzelnen Teilströme bestimmt wird. Mit der Messung gemäß diesem Verfahren wird die elektrostatische Aufladung der Pigmente in Bezug auf das polymere Bindemittelsystem bestimmt und die Stabilität der Dispersion, nicht aber deren Dispersionsgrad, charakterisiert.

Aus dem Journal Dispersion Science and Technology, 7 (2), Seite 159 bis 185 (1986), ist ein Verfahren zur Bestimmung des Dispersionsgrades von magnetischen Dispersionen bekannt, bei dem aus der Aufnahme von Quecksilber an der Oberfläche der magnetischen Pigmente auf deren Volumen geschlossen wird. Diese Methode ist zu aufwendig und als Schnellprüfung ungeeignet.

In der EP 0 103 655 wird die durch ein Rohr strömende Dispersion einem magnetischen Wechselfeld variabler Frequenz ausgesetzt und daraus die Susceptibilität bestimmt. Diese Bestimmungsmethode ist stark abhängig von der Koerzitivkraft der magnetischen Pigmente sowie der Durchströmgeschwindigkeit und ist daher als universelle Meßmethode im Produktionsbetrieb ungeeignet. Eine ähnliche Vorrichtung ist in der japanischen Anmeldung 58-76758 beschrieben.

In der DE-OS 29 29 018 ist eine Einrichtung für die Messung der Aggregation von Partikeln mit einer Wand oder untereinander beschrieben, wobei die Partikel in einer Flüssigkeits- oder Gasströmung dispergiert sind. Das flüssige oder gasförmige Mehrphasensystem wird auf eine durchsichtige Wand gerichtet und beleuchtet. Das gestreute, reflektierte oder durch Absorption geschwächte Licht wird auf einen Detektor gerichtet und ausgewertet. Die Messung der Aggregation der Partikel untereinander oder an der Wand funktioniert nur bei stark verdünnten Dispersionen, die transparent sind. Sie ist nicht geeignet für hochkonzentrierte Dispersionen welche magnetische Partikel enthalten, weil diese opak sind.

Aus der US 4 449 821 ist eine Vorrichtung zur Messung der Farbe einer flüssigen Dispersion bekannt, bei der eine Lichtquelle die Wand einer Zelle beleuchtet, hinter der die Dispersion vorbeifließt und wobei das von der Probe reflektierte Licht einem Beugungsgitter zugeführt wird, welches das Licht in einzelne Wellenlängen teilt, wobei eine Anordnung von Fotozellen die Spektralanteile messen und das von ihnen ausgehende elektrische Signal farbmetrisch beurteilt wird. Ein Teil des von der Lichtquelle ausgehenden Lichtes wird in einem Strahlenteiler abgelenkt und dieser Referenzstrahl wird von rot- und blauempfindlichen Sensoren abgetastet, und das erhaltene Referenzsignal wird bei der Farbmetrischen Auswertung berücksichtigt.

In dem Artikel "$Cr_2O_3$-Pulver mit unterschiedlicher Teilchengrößenverteilung, Autor Professor Geismar, veröffentlicht in Farbe + Lack (1982),wird eine farbmetrische Auswertung von Chromgrün beschrieben, welche eine nahezu lineare Beziehung zwischen der relativen Farbstärke und dem Produkt aus dem häufigsten Volumendurchmesser und der Standardabweichung der Verteilungskurve erkennen läßt".

Aus der DIN 53 238 Teil 22 ist es weiterhin bekannt, das Dispergierverhalten von Buntpigmenten beim Dispergieren anhand der Farbstärkeentwicklung zu bewerten.

Aus DIN 53 236 und DIN 6174 in Verbindung mit DIN 5033 ist die Farbmetrische Bestimmung von Farbabständen bei Körperfarben nach der CIELAB-Formel bekannt.

Obige DIN Normen sind z.B dem DIN Taschenbuch 49, Farbmittel 1, Berlin, 1985 (ISBN 3-410-11771-7) zu entnehmen.

Aufgabe der Erfindung war es daher, für die Kontrolle des Dispersionsgrades der magnetischen Pigmente in einer Dispersion ein eindeutiges Verfahren anzugeben, das darüber hinaus auch geeignet ist, Basis für eine kontinuierliche Messung und Überwachung der relevanten Eigenschaften der Dispersion im Produktionsprozeß zu sein und das eine schnelle und genaue Kontrollmethode darstellt.

Erfindungsgemäß wurde die Aufgabe gelöst durch ein Verfahren mit den im Anspruch 1 genannten Merkmalen. Weitere Einzelheiten der Erfindung gehen aus den Unteransprüchen, der Beschreibung und den Zeichnungen hervor.

Die Erfindung wird nun anhand der Zeichnungen näher geschildert und zwar zeigen

Figur 1, a – c

drei verschiedene schematische Beleuchtungs- und Meßgeometrien zur Bestimmung des von einer Probe remittierten Lichtes gemäß dem Stand der Technik

Figur 2

das Schema eines bevorzugten erfindungsgemäßen Verfahrens zur Kontrolle des Dispersionsgrades

Figur 3

ein Ausführungsbeispiel der Probenkontrolle mit dem erfindungsgemäßen Verfahren

Figur 4

die schematische Darstellung des CIE-LAB-Farbsystems

Figuren 5 – 6

Diagramme mit der relativ zur Dispergierfunktion gemessenen Farb-änderung an Dispersionen.

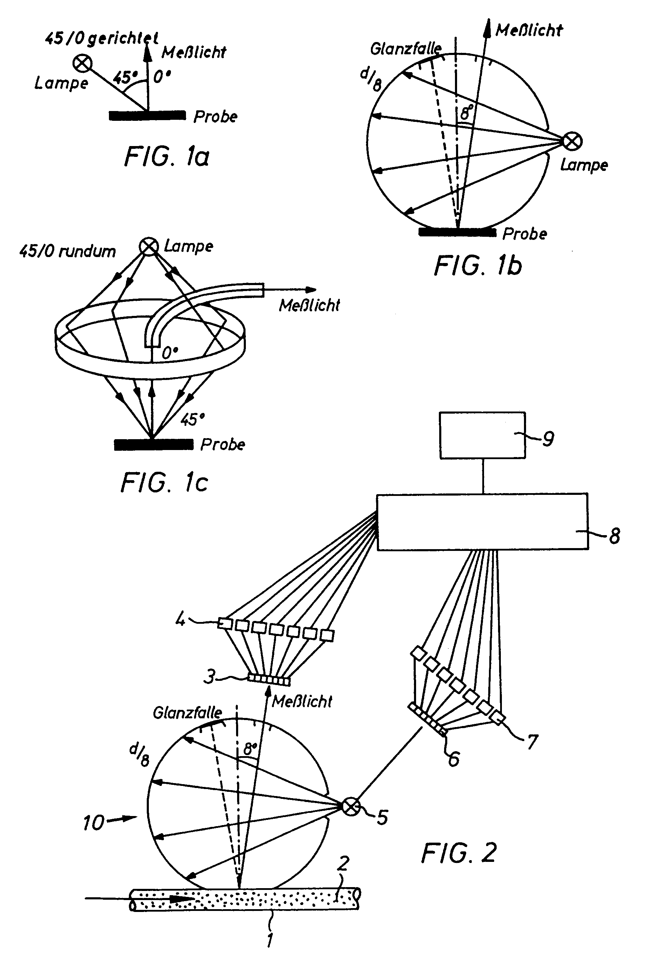

Ein wesentliches Merkmal der Erfindung besteht darin, daß die Dispersion beleuchtet wird und das von der Dispersion remittierte Licht farbmetrisch bewertet und mit einem Standardwert verglichen wird. Farbmetrische Methoden zur Bestimmung einer gewünschten Produktqualität sind beispielsweise aus der Textilfärbetechnik oder bei der Lackierung von Gegenständen, also bei festen Stoffen, bekannt. Figur 1 zeigt drei verschiedene übliche Meßgeometrien, wie sie in DIN 5033, Teil 7, festgelegt sind.

a) Die Probe wird unter 45° Auftreffwinkel zur Senkrechten bestrahlt und das bei 0° remittierte Licht wird gemessen.

b) Die Probe wird diffus durch eine Hohlkugel, die innen mit einem mattweißen Belag versehen ist, beleuchtet, und das unter 8° zur Senkrechten remittierte Licht wird gemessen. Dabei kann eine schwarze Glanzfalle zur Eliminierung des Glanzlichtes eingeschaltet werden.

c) Die Probe wird rundum unter 45° beleuchtet und das bei 0° zur Senkrechten remittierte Licht wird gemessen.

Es wurde gefunden, daß die unter b) beschriebene Methode am besten für den vorgesehenen Zweck geeignet war.

Figur 2 stellt ein erfindungsgemäßes Verfahren schematisch dar. Die durch ein Rohr (1) mit transparenten, möglichst entspiegelten Wänden fließende magnetische Dispersion (2) wird wie oben dargestellt diffus beleuchtet. Das remittierte Licht wird spektral zerlegt, beispielsweise mit einem holographischen Gitter (3), und das zerlegte Licht wird beispielsweise mit je 20 nm Wellenlängenabstand im Gebiet von 400 bis 700 nm mit einer entsprechenden Anzahl Photodioden (4) gemessen. Analog dazu wird das von der Lichtquelle (5) ausgehende Licht spektral zerlegt (6) und ebenso wie das remittierte Licht gemessen (7). Bevorzugt verwendet wurde eine Anordnung von zwei gepulst betriebenen Xenon–Blitzlampen im Zweistrahlverfahren. Die farbmetrische Bewertung wird weiter unten beschrieben.

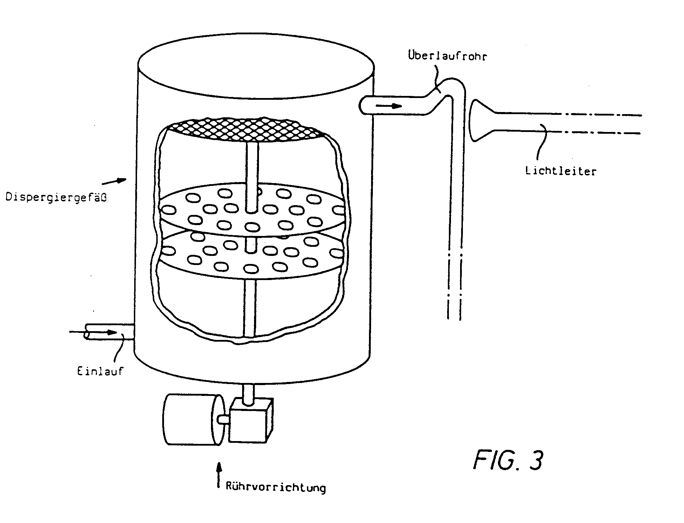

In dem oben beschriebenen Fall wird an einer Meßstelle die Bestimmung des Dispersionsgrades kontinuierlich durchgeführt. Sollen jedoch mehrere Meßstellen gleichzeitig verfolgt werden, was bei der oben beschriebenen Feindispergierung im Produktionsbetrieb vonnöten sein kann, so können als Meßstellen jeweils das Überlaufrohr von einem Dispergiergefäß zum nächsten als Meßstelle verwendet werden, wobei zur zentralen Beleuchtungsvorrichtung und zur Ausmessung beziehungsweise Bewertung jeweils taktmäßig zuschaltbare Lichtleiter eingesetzt werden, wie in Figur 3 schematisch dargestellt. Konkrete Ausführungsformen des erfindungsgemäßen Verfahrens sind in den Beispielen aufgeführt.

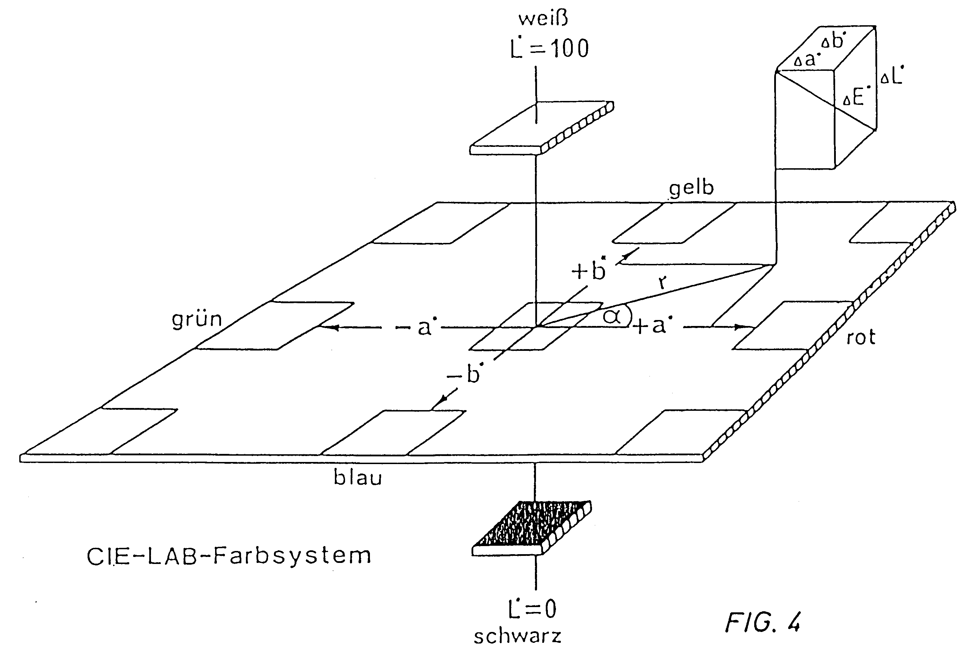

Das Prinzip der farbmetrischen Messung besteht, wie jedem Fachmann auf dem betreffenden Gebiet bekannt, darin, das von einer Probe ausgehende Licht spektral bei bestimmten Wellenlängen, den sogenannten Auswahlwellenlängen, zu messen, die Meßwerte in farbphysiologische Funktionen einzusetzen und daraus für eine bestimmte Normlichtart den Farbton, die Farbsättigung und die Helligkeit zu berechnen. Bei der Darstellung der berechneten Werte hat sich weitgehend das CIE–LAB–Farbsystem nach DIN 6174 (Figur 4) durchgesetzt, bei dem die Farbwerte $(\Delta a^*, \Delta b^*)$ und die Helligkeitswerte $(\Delta L^*)$ ausgehend vom Unbuntpunkt in der Mitte des Diagramms vektoriell zu dem Endwert, dem Farbabstand $\Delta E^*$ zusammengesetzt werden.

$$\Delta E^* = \sqrt{(\Delta L^*)^2 + (\Delta a^*)^2 + (\Delta b^*)^2}$$

Im vorliegenden Fall wird in der Recheneinheit 8 (Figur 2) das von der Lichtquelle (5) beziehungsweise von der Probe, der Dispersion (2) kommende spektral zerlegte (3, 6) und durch die Photodioden (4, 7) gemessene Licht bewertet und daraus der Farbabstand $\Delta E^*$, also der Farb– abstand zwischen Lichtquelle (Bezug) und dem von der Probe remittierten Licht berechnet und angezeigt, beispielsweise digital oder in einer Graphik auf einem Bildschirm (9). Derartige Geräte sind auf dem Markt erhältlich und haben Meß– und Rechenzeiten < 1 Sekunde, so daß die gewünschten Werte außerordentlich schnell verfügbar sind.

Dabei wurde, wie in den folgenden Beispielen gezeigt wird, gefunden, daß die Farb änderungen während des Grob– und Feindispergierens außerordentlich empfindlich den Dispersiongrad der magnetischen Pigmente wiedergeben, wesentlich genauer als die bisher bekannten oben aufgeführten Meßmethoden, so daß das angestrebte Ziel, nämlich eine genaue Qualitätskontrolle der Dispersion, erreicht werden kann. Der gewünschte Endwert ist dann gegeben, wenn ein aufgrund einer Bezugsprobe (Standard) farbmetrisch gemessener und festgelegter Farbabstand erreicht ist.

Beispiel 1 (alle Angaben beinhalten Gewichtsteile)

Chargenweise wurden jeweils 1 200 Teile nadelförmiges Co–dotiertes $\gamma$ – $Fe_2O_3$ mit einer Koerzitivfeldstärke von 27 kA/m und einer Sättigungsmagnetisierung von 1,5 T mit 105 Teilen Copolymerisat Vinylchlorid/Vinylacetat/Vinylalkohol, 128 Teilen Polyester–Polyurethan, 30 Teilen saures Alkylphosphat als Dispergierungsmittel, 3 Teilen Fettsäure und 12 Teilen Zinkoleat in 2 250 Teilen Lösungsmittelgemisch Tetrahydrofuran/Cyclohexanon in einem Gefäß mit einem Turbomixer 5 Stunden lang vordispergiert. Die so hergestellte Dispersion wurde in ein Zwischengefäß mit langsam laufenden Blattrührern gepumpt. Aus diesem wurde die Dispersion kontinuierlich durch eine Reihe von sechs aufeinanderfolgenden Rührwerkskugelmühlen zur Feindispergierung gepumpt, welche jeweils 125 l Volumen hatten und mit 91 l Aluminiumoxid–Mahlkugeln von 1 – 1,5 mm Durchmesser gefüllt waren. Die Verweilzeit der Dispersion in jeder Mühle betrug 30 Minuten. Am Eingang der ersten Mühle (Meßstelle 0) sowie am Auslauf jeder Mühle (Meßstellen 1 bis 6) befanden sich jeweils Meßstellen für die erfindungsgemäße Anordnung, die Übertragung des Meßlichtes sowie des remittierten Lichtes geschah über 4,5 m lange Lichtleiter. Die aus der letzten Mühle der Kaskade austretende Dispersion wurde unter Druck durch einen Filter mit 1 $\mu m$ Porenweite filtriert, mit 0,85 Teilen Vernetzer (Desmodur L der Bayer AG) pro 100 Teilen Dispersion, gelöst in einem Teil Tetrahydrofuran Lösungsmittel, versetzt und mittels eines Extrudergießers auf einer 16 $\mu m$ dicken Polyethylenterephthalatfolie mit einer Trokkendicke von 3 $\mu m$ aufgetragen unter gleichzeitiger Ausrichtung der magnetischen Pigmente mittels eines Permanentmagneten. Die trockene Schicht wurde durch Hindurchführen zwischen beheizten Walzen unter Druck geglättet.

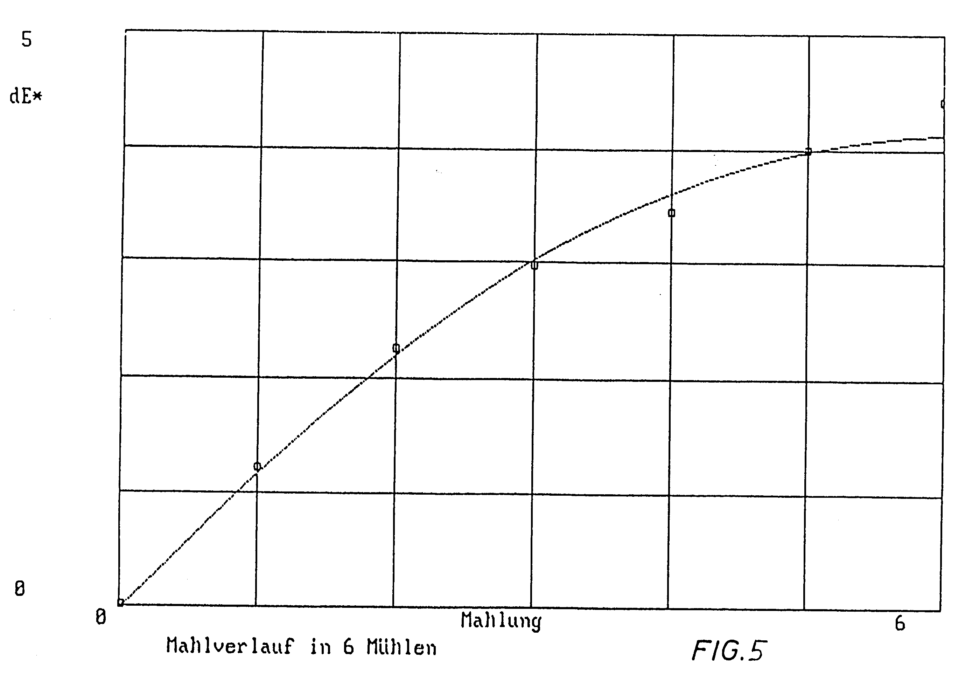

Die Bewertung der einzelnen Meßstellen ergab als Resultat das in Figur 5 gezeigte Diagramm, bei dem die $\Delta E^*$–Werte gegen die einzelnen Meßstellen aufgetragen sind. Dabei wurde willkürlich der jeweilige Meßwert der Meßstelle 0 = 0 gesetzt. Der kurvenmäßige Verlauf kann mathematisch durch Vorgabe eines Polynoms n–ten Grades oder einer logarithmischen Funktion über eine Regressionsrechnung von der Recheneinheit dargestellt werden.

Als Resultat zeigten sich hervorragende elektroakustische und mechanische Daten des so hergestellten magnetischen Aufzeichnungsträgers, während Proben, die lediglich 1 – 5 Mühlen durchlaufen hatten, in den Eigenschaften unterlegen waren.

Beispiel 2

737 Teile nadelförmiges $CrO_2$ mit 39,8 kA/m Koerzitivkraft wurden zusammen mit 25 Teilen Vinylidenchlorid/Acrylnitril – Mischpolymerisat, 23 Teilen Lecithin, 580 Teilen Tetrahydrofuran und 209 Teilen Cyclohexanon als Lösungsmittel dispergiert und zu einer Bindemittellösung, bestehend aus 27 Teilen Vinylidenchlorid/Acrylnitril – Mischpolymerisat, 106 Teilen Polyesterurethan in 534 Teilen Tetrahydrofuran und 200 Teilen Cyclohexanon zugegeben. Die so hergestellte Dispersionscharge wurde in einem Gefäß 6 Stunden mit einem Turbomixer vordispergiert, in ein Zwischengefäß gepumpt und aus diesem kontinuierlich durch eine Reihe von sieben aufeinanderfolgenden Rührwerkskugelmühlen zur Feindispergierung gepumpt, welche jeweils 125 l Volumen hatten und mit Keramik – Mahlkörpern von 1 bis 1,5 mm Durchmesser zu 80 % gefüllt waren. Die Verweilzeit in jeder Mühle betrug 25 Minuten. Danach wurde die feindispergierte Dispersion durch einen Filter mit 1 $\mu$m Probenweite gepreßt, mit 4 Teilen Fettsäure, 9 Teilen Butylstearat und 15 Teilen einer 75%igen Lösung in Ethylacetat eines Vernetzers (Desmodur L der Bayer AG) mit 0,2 Teilen Eisenacetylacetonat gemischt und in einem Extrudergießer auf eine 8 $\mu$m PET – Folie mit einer Trockenschichtdicke von 3 $\mu$m vergossen, im Magnetfeld ausgerichtet, getrocknet und kalandriert.

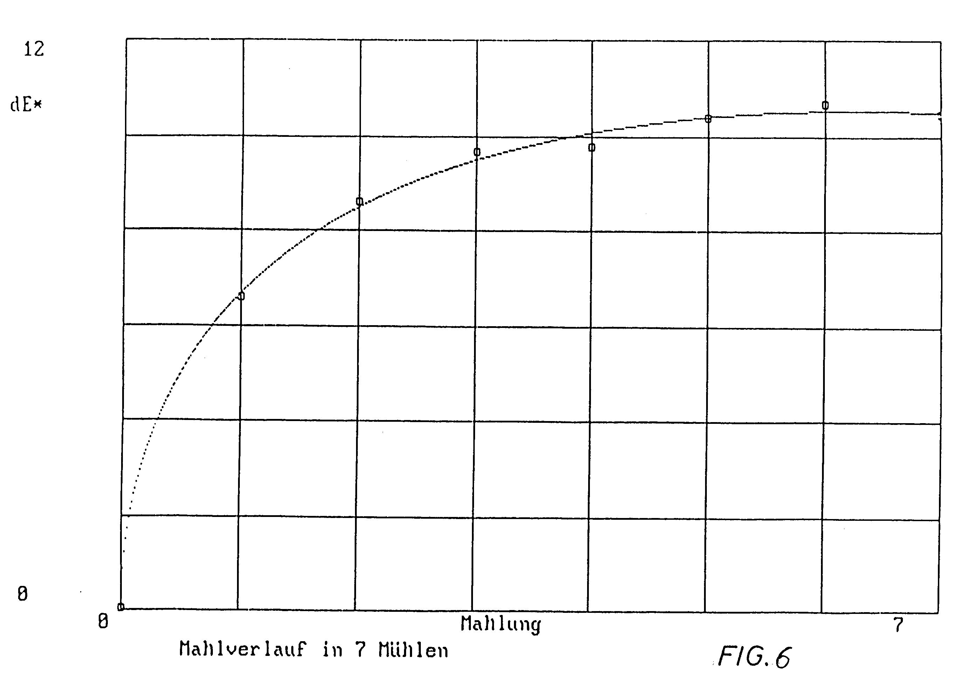

Die Kontrolle des Dispersionsgrades mittels des erfindungsgemäßen Verfahrens sowie die parallel dazu erfolgende Entnahme von Dispersionsproben geschah analog zu Beispiel 1. Figur 6 zeigt als Diagramm $\Delta E^*$ als Maß für den Dispersionsgrad bei den sieben Mühlen, wobei der Meßwert beim Einlauf in die erste Mühle wieder gleich Null gesetzt wurde.

Die Vorteile des erfindungsgemäßen Verfahrens lassen sich wie folgt zusammenfassen:

– Schnelle und genaue Kontrolle des Dispersionsgrades der magnetischen Pigmente von Dispersionen im flüssigen Zustand oder bei vergossenen Proben im trockenen Zustand.

– Kontinuierliche Verfolgung des Dispersionsgrades während der Vor – und Feindispergierung in kontinuierlich betriebenen Fertigungsanlagen.

– Dispergierapparaturfehler beziehungsweise Fehler in der Mühlenkaskade können sofort erkannt, lokalisiert und entsprechend gegengesteuert werden, so daß kein Produktionsausfall eintritt.

– Der Ablauf der Dispersionsgradbestimmung kann weitgehend automatisiert werden, so daß nur ein geringer Überwachungsaufwand besteht.

**Patentansprüche**

1. Kontrollverfahren bzgl. des Dispersionsgrades der ferromagnetischen Pigmente bei der Dispergierung einer Dispersion, welche zur Herstellung von magnetischen Aufzeichnungsträgern geeignet ist und aus einer polymeren Bindemittellösung, darin dispergierten ferromagnetischen Pigmenten und weiteren Zusatzstoffen zusammengesetzt ist, wobei entweder einer Mühle in zeitlichen Abständen eine Stichprobe der Dispersion mit besagten Eigenschaften entnommen, auf einen transparenten Schichtträger vergossen und getrocknet wird und eine oder mehrere Lagen von Schichtproben übereinanderliegend als Probe bestrahlt wird oder als Probe die in einem transparenten Überlaufrohr der Mühle vorbeiströmende Dispersion selbst bestrahlt wird, wobei die Probe mit einer Beleuchtungs – und Abbildungseinrichtung bestrahlt und im Zweistrahlverfahren das von der Probe remittierte Licht sowie die Lichtquelle in einem Detektor bezüglich Farbe und/oder Helligkeit gemessen und mit einer Auswertevorrichtung angezeigt wird, wobei die Probe (2) diffus durch eine von einer gepulst betriebenen Xenonlampe (5) beaufschlagten Hohlkugel (10), die innen mit einem mattweißen Belag versehen ist, beleuchtet und das unter 8° zur Senkrechten remittierte Licht sowie das direkt von der Xenonlampe (5) ausgehende Licht jeweils Beugungsgittern (3, 6) zugeführt wird, die das auftreffende Licht spektral zerlegen und Fotozellen – Anordnungen (4, 7) zuführen, wobei das von der Probe (2) remittierte beziehungsweise das von der Lichtquelle ausgehende Licht in einem Rechner in farbmetrische Werte umgerechnet wird und daraus der Farbabstand $\Delta E^*$ des Probenlichtes vom Unbuntpunkt nach der CIELAB – Formel

$$\Delta E^* = \sqrt{(\Delta L^*)^2 + (\Delta a^*)^2 + (\Delta b^*)^2}$$

bestimmt und angezeigt wird und wobei die Feinmahlung beendet wird, wenn ein aufgrund einer Bezugsprobe (Standard) farbmetrisch gemessener und festgelegter Farbabstand $\Delta E^*$ erreicht ist.

**2.** Verfahren nach Anspruch 1, dadurch gekenn – zeichnet, daß die Messung mit einem Zweistrahl – Spektral – Fotometer mit zwei ge – pulsten Xenon – Lampen geschieht.

## Claims

**1.** A method for monitoring the dispersivity of ferromagnetic pigments during the dispersing of a dispersion which is suitable for the pro – duction of magnetic recording media and is composed of a polymeric binder solution, fer – romagnetic pigments dispersed therein and further additives, wherein either a sample of the dispersion having said properties is taken from a mill at intervals of time, cast on a transparent substrate and dried and one or more strata of layer samples one on top of the other as a sample is exposed to light or the dispersion itself flowing past in a transparent overflow pipe of the mill is used as the sample and is exposed to light, the sample is exposed using an illuminating and focusing means and the light reflected by the sample and the light source are measured in a detector with regard to color and/or lightness by the two – beam method and displayed by means of an evalu – ating apparatus, the sample (2) is illuminated diffusely through a hollow sphere (10) which receives light from a pulsed xenon lamp (5) and is provided on the inside with a matt white coating and the like are selected at 8° to the vertical and the light emanating directly from the xenon lamp (5) are each passed to dif – fraction gratings (3, 6) which divide the in – cident light into its spectral components and pass it to photocell arrangements (4, 7), the light reflected by the sample (2) and the light emanating from the light source are converted into colorimetric values in a computer and, from these values, the color difference $_DE^*$ of the sample light from the achromatic point is determined according to the CIELAB formula

$$\Delta E^* = \sqrt{(\Delta L^*)^2 + (\Delta a^*)^2 + (\Delta b^*)^2}$$

and is displayed, and fine milling is terminated when a color difference $_DE^*$ determined and measured colorimetrically on the basis of a reference sample (standard) has been reached.

**2.** A method as claimed in claim 1, wherein the measurement is carried out using a two – beam spectrophotometer with two pulsed xenon lamps.

## Revendications

**1.** Procédé de contrôle du degré de dispersion des pigments ferromagnétiques lors de la dispersion d'une dispersion qui convient à la fabrication de supports magnétiques d'enre – gistrement et est composée d'une solution de liant polymère, de pigments ferromagnétiques dispersés dans celle – ci et d'autres additifs, dans lequel, ou bien un échantillon de la dis – persion ayant lesdites propriétés est prélevé par intervalles dans un broyeur, coulé sur un substrat transparent et séché, et une ou plu – sieurs couches d'échantillons en couche sont irradiées superposées comme échantillon, ou bien la dispersion passant dans un tuyau de trop – plein du broyeur elle – même est irradiée comme échantillon, où l'échantillon est irradié avec un dispositif d'éclairage et de formation d'image et la lumière renvoyée par l'échantil – lon et la source lumineuse sont mesurées en ce qui concerne la couleur et/ou la luminance dans un détecteur par le procédé à double faisceau et indiquées par un dispositif d'ex – ploitation, l'échantillon (2) est éclairé de ma – nière diffuse à travers une sphère creuse (10) où agit une lampe au xénon (5) pulsée et qui est pourvue intérieurement d'un revêtement blanc mat, et la lumière renvoyée à 8 degrés de la normale et la lumière émanant directe – ment de la lampe au xénon (5) sont envoyées chacune à un réseau de diffraction (3, 6) qui décompose la lumière qu'il reçoit et l'envoie à un dispositif à cellules photo – électriques (4, 7), la lumière renvoyée par l'échantillon (2) et la lumière émanant de la source lumineuse sont converties dans un calculateur en valeurs colorimétriques et de là, l'écart colorimétrique deltaE* de la lumière de l'échantillon du point achromatique est déterminé par la formule CIE – LAB

$$\Delta E^* = \sqrt{(\Delta L^*)^2 + (\Delta a^*)^2 + (\Delta b^*)^2}$$

et indiqué, et il est mis fin au broyage fin lorsqu'un écart colorimétrique deltaE* mesuré et fixé colorimétriquement sur la base d'un échantillon de référence (étalon) est atteint.

**2.** Procédé selon la revendication 1, caractérisé par le fait que la mesure est faite avec un spectrophotomètre à double faisceau à deux lampes au xénon pulsées.

45/0 gerichtet
Meßlicht
Lampe
45° 0°
Probe

FIG. 1a

Meßlicht
Glanzfalle
d/8
8°
Lampe
Probe

FIG. 1b

45/0 rundum
Lampe
Meßlicht
0°
45°
Probe

FIG. 1c

9
8
4
3
Meßlicht
Glanzfalle
d/8
8°
7
6
Glanzfalle
d/8
8°
Meßlicht
10
5
2
1

FIG. 2

Überlaufrohr

Lichtleiter

Dispergiergefäß

Einlauf

Rührvorrichtung

FIG. 3

$\Delta L^{\bullet}$

$\Delta E^{\bullet}$

$\Delta b^{\bullet}$

$\Delta a^{\bullet}$

rot

gelb

weiß
$L^{\bullet}=100$

$r$

$\alpha$

$+a^{\bullet}$

$+b^{\bullet}$

$-a^{\bullet}$

$-b^{\bullet}$

grün

blau

schwarz
$L^{\bullet}=0$

CIE-LAB-Farbsystem

FIG. 4

FIG.5

Mahlverlauf in 6 Mühlen

EP 0 308 741 B1

FIG.6

Mahlverlauf in 7 Mühlen

Mahlung

dE*